# DEMANDE DE BREVET EUROPEEN

(11) **EP 3 751 198 A1**
(43) Date de publication de la demande: **16.12.2020**
(21) Numéro de dépôt: 20189049.8
(22) Date de dépôt: 31.05.2016
(51) Int. Cl.: F23G 7/06, H05B 3/40, F23G 5/24, F23C 3/00, F23C 99/00, B01J 19/24, C10G 9/20, C10K 3/00

(54) **FOUR DE CRAQUAGE**

(30) Priorité: 05.06.2015 FR 1555148; 15.09.2015 FR 1558609
(62) Demande divisionnaire de: 16726560.2
(71) Demandeur: E.T.I.A.-Evaluation Technologique, Ingenierie et Applications, 60201 Compiegne (FR)
(72) Inventeur: LEPEZ, Olivier, 60260 LAMORLAYE (FR); SAJET, Philippe, 60610 LACROIX-SAINT-OUEN (FR)
(74) Mandataire: Decorchemont, Audrey Véronique Christèle

(57) **Abrégé**

L'invention concerne un four de craquage (1) comportant une enceinte (2) tubulaire verticale qui comprend une entrée (4) pour l'introduction d'un gaz à traiter et une sortie (5) pour l'évacuation dudit gaz hors de l'enceinte, des moyens de chauffage dudit gaz qui comprennent un tube de chauffage (9) s'étendant verticalement à l'intérieur de l'enceinte et coaxialement à l'enceinte, le tube de chauffage étant conformé de sorte à avoir son extrémité inférieure fermée et étant agencé de sorte que son extrémité inférieure soit agencée dans l'enceinte et de sorte que son extrémité supérieure soit raccordée à un brûleur (13) des moyens de chauffage qui sont agencés à l'extérieur de l'enceinte. L'entrée (4) est agencée de sorte à faire pénétrer le gaz à traiter dans l'enceinte (2) le long de la paroi interne de l'enceinte (2) afin de générer un effet cyclonique de sorte que le gaz s'écoule hélicoïdalement dans l'enceinte.

## Description

L'invention concerne un four de craquage pour le traitement d'un gaz notamment, bien que non exclusivement, d'un gaz issu d'un traitement thermique de déchets et/ou de biomasses. L'invention concerne également un ensemble comportant un tel four de craquage et un dispositif de traitement thermique de biomasses et/ou de déchets dont une sortie est raccordée à l'entrée dudit four de craquage.

Pour les biomasses, il peut s'agir de fractions biodégradables de produits, déchets et résidus provenant de l'agriculture, de la sylviculture et des industries connexes, avec en particulier des biomasses d'origine végétale ou des parties solides de boues de station d'épuration, ainsi que les fractions biodégradables des déchets industriels et municipaux. Pour les déchets, il peut s'agir de déchets industriels en particulier des déchets polymériques (matières plastiques, caoutchoucs, ...).

### ARRIERE-PLAN TECHNOLOGIQUE

Dans un souci économique et écologique, il est de plus en plus courant de traiter les biomasses et/ou les déchets en vue de l'obtention de matières combustibles (solide, liquide ou gaz) à vocation énergétique.

Par exemple il a été proposé de valoriser des biomasses et/ou des déchets afin de les transformer en gaz utilisable par un moteur à gaz. A cet effet, un traitement thermique (pyrolyse, gazéification ...) des biomasses et/ou des déchets permet de récupérer un gaz à haute valeur énergétique. Toutefois le gaz ainsi récupéré s'avère trop pollué par des goudrons ou des phases huileuses pour pouvoir être utilisé sans risque par un moteur à gaz.

Pour pallier cet inconvénient, il est connu de faire succéder à l'étape de traitement thermique des biomasses et/ou des déchets, une étape de craquage. Ceci permet de craquer les goudrons et les phases huileuses de sorte à récupérer en sortie de l'étape de craquage un gaz suffisamment propre pour pouvoir être utilisé par un moteur à gaz (moyennant éventuellement une ou des étapes supplémentaires de purification pour des composants parasites autres que les goudrons et les phases huileuses).

Il a par exemple été proposé de mettre en œuvre l'étape de craquage à l'aide d'une torche à plasma. Toutefois, une telle solution s'avère relativement onéreuse.

Ainsi, une solution moins coûteuse a été proposée qui consiste à faire circuler le gaz à traiter dans une conduite traversant une enceinte chauffée intérieurement à l'aide d'un gaz de chauffage. Le chauffage indirect de la conduite par le gaz de chauffage entraîne alors une réaction de craquage des goudrons et des phases huileuses contenus dans le gaz.

Néanmoins, il a été constaté qu'une telle solution ne permettait pas une purification très poussée en goudrons et phases huileuse du gaz à traiter.

### OBJET DE L'INVENTION

Un but de l'invention est de proposer un four de craquage permettant une meilleure élimination des goudrons et/ou des phases huiles contenus dans un gaz à traiter. Un but de l'invention est également de proposer un ensemble comportant un tel four de craquage et un dispositif de traitement thermique de biomasses et/ou de déchets dont une sortie est raccordée à l'entrée dudit four de craquage.

### DEFINITION GENERALE DE L'INVENTION

Le problème précité est résolu conformément à l'invention grâce à un four de craquage comportant une enceinte tubulaire verticale qui comprend une entrée pour l'introduction d'un gaz à traiter et une sortie pour l'évacuation dudit gaz hors de l'enceinte, des moyens de chauffage dudit gaz qui comprennent un tube de chauffage s'étendant verticalement à l'intérieur de l'enceinte et coaxialement à l'enceinte, le tube de chauffage étant conformé de sorte à avoir son extrémité inférieure fermée et étant agencé de sorte que son extrémité inférieure soit agencée dans l'enceinte et de sorte que son extrémité supérieure soit raccordée à un brûleur des moyens de chauffage qui est agencé à l'extérieur de l'enceinte, dans lequel l'entrée est agencée de sorte à faire pénétrer le gaz à traiter dans l'enceinte le long de la paroi interne de l'enceinte afin de générer un effet cyclonique de sorte que le gaz s'écoule hélicoïdalement dans l'enceinte.

Ainsi, l'agencement particulier de l'enceinte et du tube de chauffage permet de créer une zone de traitement dans laquelle le gaz est bien confiné. Ceci permet de favoriser les échanges thermiques au sein de l'enceinte entre le gaz à traiter et le tube de chauffage : le craquage des goudrons et phases huiles s'avère donc efficace et rapide de telle sorte que le gaz récupéré en sortie de four de craquage présente une bonne pureté.

En particulier, dans le cas où le gaz à traiter est un gaz issu d'un traitement thermique (pyrolyse, gazéification ...) de biomasse et/ou de déchets, le gaz récupéré en sortie du four de craquage selon l'invention est suffisamment exempt de goudrons et de phases huileuses pour pouvoir être utilisé dans un moteur à gaz.

Bien entendu, dans la présente demande les termes « supérieur », « inférieur », « horizontal », « vertical » ... doivent s'entendre selon le sens d'utilisation du four à craquage c'est-à-dire lorsque le brûleur se trouve à l'aplomb du reste de l'enceinte.

Dans la présente demande, le terme de céramique doit s'entendre comme étant un matériau réfractaire et inerte (i.e. qui n'est pas ou peu sensible au contact d'un gaz à traiter même corrosif), ledit matériau étant manufacturé et inorganique. Un métal ou un alliage n'est donc pas une céramique au sens de l'invention tout comme un béton.

L'invention concerne également un ensemble d'un four de craquage ainsi décrit et d'un dispositif de traitement thermique de biomasses et/ou de déchets dont une sortie est raccordée à l'entrée dudit four de craquage.

D'autres caractéristiques et avantages de l'invention apparaîtront plus clairement à la lumière de la description qui va suivre et des dessins annexés, concernant des modes de réalisation particuliers de l'invention.

### BREVE DESCRIPTION DES DESSINS

Il sera fait référence aux figures annexées ci-jointes parmi lesquelles :
- la figure 1 est une vue schématique d'un four à craquage selon un premier mode de réalisation de l'invention,
- la figure 2 est une vue schématique d'un four à craquage selon un deuxième mode de réalisation de l'invention.

### DESCRIPTION DETAILLEE DE MODES DE REALISATION PARTICULIERS DE L'INVENTION

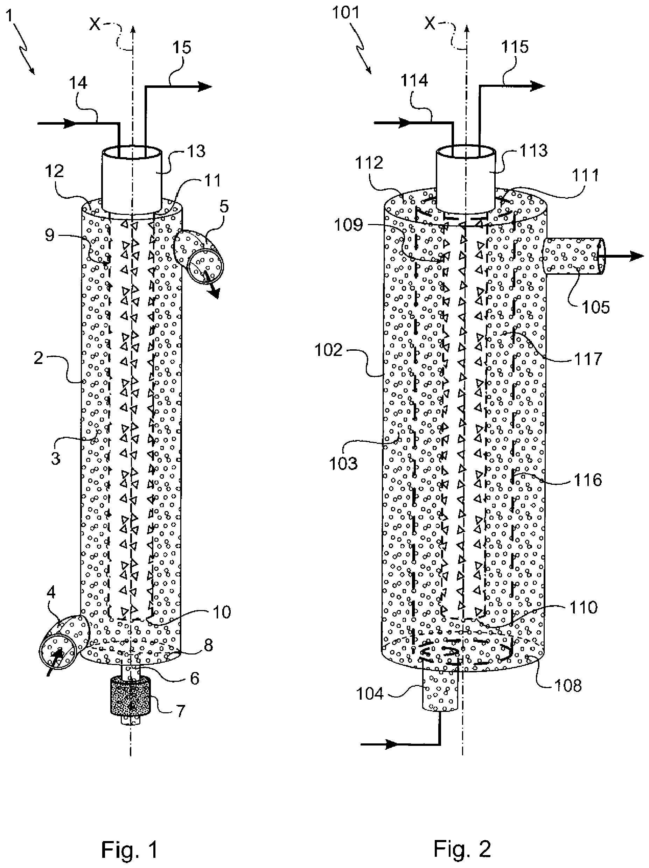

En référence à la figure 1, le four de craquage selon le premier mode de réalisation de l'invention, généralement désigné par 1, comporte une enceinte 2.

De préférence, le four à craquage 1 permet la mise en œuvre d'une étape de craquage pour un gaz (symbolisé par des ronds à la figure 1) issu d'un traitement thermique de biomasses et/ou de déchets (par exemple pyrolyse ou gazéification), ledit gaz comprenant goudrons et phases huileuses à éliminer par ladite étape de craquage. Le four de craquage 1 est donc ici conformé pour soumettre le gaz à une température comprise entre 900°C (degrés Celsius) et 1500°C, et de préférence entre 1000°C et 1300°C. Le four de craquage 1 est préférentiellement conformé pour soumettre le gaz à une température d'environ 1100°C. Par ailleurs, le four de craquage 1 est ici conformé pour que le gaz traverse l'enceinte 2 avec un temps de séjour court (typiquement entre 0,5 et 2 secondes).

L'enceinte 2 est conformée en une enceinte 2 tubulaire d'axe X vertical. L'enceinte 2 est ici également conformée de sorte à présenter une section circulaire (section ayant pour normale l'axe X).

L'enceinte 2 comporte une entrée 4 pour l'introduction du gaz à traiter, entrée 4 qui est ici raccordée au dispositif de traitement thermique des biomasses et/ou des déchets qui ont permis la génération dudit gaz à traiter. Un tel dispositif comporte par exemple un bâti et des moyens de convoyage des biomasses et/ou des déchets entre l'entrée et la sortie du bâti qui comprennent une vis montée pour tourner dans le bâti selon un axe géométrique de rotation et des moyens d'entraînement en rotation de la vis, le dispositif comportant en outre des moyens de chauffage de la vis par effet Joule. Un tel dispositif est notamment décrit dans les documents WO 99/39549 et FR 2 892 888.

L'enceinte 2 comporte par ailleurs une sortie 5 par laquelle est évacué le gaz. L'entrée 4 est agencée en partie basse de l'enceinte 2 et la sortie 5 est agencée en partie haute de l'enceinte. De façon particulière, l'entrée 4 s'étend sensiblement tangentiellement à l'enceinte 2. L'entrée 4 est donc agencée de sorte à faire pénétrer le gaz dans l'enceinte 2 le long de la paroi interne de l'enceinte 2. Ceci permet de générer un effet cyclonique de sorte que le gaz s'écoule hélicoïdalement dans l'enceinte 2. Ceci favorise le traitement du gaz.

De préférence, la sortie 5 est également agencée tangentiellement à l'enceinte 2. De préférence, l'entrée 4 et la sortie 5 sont agencées à l'opposée l'une de l'autre, selon la direction radiale, relativement à l'enceinte 2 afin de faciliter la circulation du gaz à traiter dans toute l'enceinte 2.

De façon privilégiée, l'enceinte 2 est en matériau réfractaire. Les parois internes de l'enceinte 2 présentent donc de bonnes propriétés de rayonnement thermique. Plus précisément ici, l'enceinte 2 est en céramique. La céramique choisie pour l'enceinte 2 présente de préférence une densité de puissance surfacique comprise entre 10 et 50 kilowatt par mètre carré. La céramique choisie est par exemple de l'alumine. Le four de craquage 1 comporte par ailleurs des moyens d'évacuation de particules solides parasites, tels que des poussières, contenues dans le gaz à traiter. A cet effet, les moyens d'évacuation comportent ici une conduite d'évacuation 6 et une vanne 7, par exemple de type rotative, guillotine ou à double sas, agencée dans ladite conduite d'évacuation 6. La vanne 7 permet d'assurer une étanchéité de l'enceinte 2 afin de limiter l'entrée d'oxygène par la conduite d'évacuation 6 dans l'enceinte 2, oxygène qui nuirait au craquage. La conduite d'évacuation 6 s'étend ici depuis le fond 8 de l'enceinte 2 vers l'extérieur de l'enceinte 2. La conduite d'évacuation 6 est ici agencée de sorte à déboucher à une extrémité sensiblement au centre dudit fond 8 dans l'enceinte 2. La conduite d'évacuation 6 s'étend ici selon l'axe X.

Il suffit donc simplement d'ouvrir la vanne 7 pour laisser tomber les particules solides parasites hors de l'enceinte 2. De préférence, le fond 8 de l'enceinte 2 est concave de sorte à former un entonnoir permettant non seulement un meilleur stockage des particules solides parasites mais également une évacuation facilitée de ces particules par la conduite d'évacuation 6 débouchant dans cet entonnoir.

De plus, le four de craquage 1 comporte des moyens de chauffage dudit gaz à traiter qui comprennent notamment un tube de chauffage 9. Le tube de chauffage 9 est conformé de sorte à s'étendre verticalement selon l'axe X dans l'enceinte 2, coaxialement à ladite enceinte 2. Le tube de chauffage 9 est ici également conformée de sorte à présenter une section circulaire (section ayant pour normale l'axe X). Ainsi, l'enceinte 2 et le tube de chauffage 9 délimitent entre eux un espace intérieur de section annulaire (section ayant pour normale l'axe X) formant une zone de traitement 3 du gaz. Par ailleurs, le tube de chauffage 9 est conformé de sorte que son extrémité inférieure 10 est fermée et agencée à l'intérieur de l'enceinte 2 sans toutefois toucher le fond 8 de l'enceinte 2. Ceci facilite le dépôt des particules solides parasites sur le fond 8 de l'enceinte 2 facilitant leur évacuation.

Le tube de chauffage 9 présente toutefois une hauteur, prise selon l'axe X, proche de celle de l'enceinte 2 typiquement comprise entre 90 et 99 % de la hauteur de l'enceinte 2. L'extrémité supérieure 11 du tube de chauffage débouche quant à elle hors de l'enceinte 2, au-dessus du plafond 12 de l'enceinte 2.

De façon privilégiée, le tube de chauffage 9 est en céramique. La céramique choisie présente de préférence une densité de puissance surfacique comprise entre 10 et 50 kilowatt par mètre carré. La céramique choisie est par exemple de l'alumine.

Les moyens de chauffage comportent en outre une conduite d'entrée 14 d'un combustible de chauffage (gaz naturel, fuel, gaz de synthèse épuré, ou encore gaz traité par le présent four de craquage 1 dont une partie est prélevée au niveau de la sortie 5 du four de craquage 1 pour alimenter la conduite d'entrée 14 ...) raccordé à un brûleur 13 desdits moyens de chauffage, brûleur 13 lui-même raccordé à l'extrémité supérieure 11 du tube de chauffage 9. Les moyens de chauffage comportent également une conduite de sortie 15 du combustible brûlé également raccordée à l'extrémité supérieure 11 du tube de chauffage 9.

De préférence, les moyens de chauffage utilisent d'abord un combustible de chauffage extérieur au four de craquage 1 pour initialiser le chauffage du tube de chauffage 9 (de type gaz naturel, fuel, gaz de synthèse épuré ...) et une fois le traitement du gaz débuté, les moyens de chauffage prélèvent une partie du gaz traité en sortie 5 du four de craquage 1 pour assurer le chauffage du tube de chauffage 9.

De la sorte, le four de craquage 1 s'avère relativement autonome et ne requiert un combustible extérieur que pour initialiser le début du craquage.

Le combustible extérieur pourra également être utilisé en cours de fonctionnement, lorsque le simple prélèvement du gaz traité en sortie 5 du four de craquage 1 n'est pas suffisant pour alimenter le brûleur.

En service, le gaz à traiter est introduit dans l'enceinte 2 par l'entrée 4. Parallèlement, le brûleur 13 assure une combustion du combustible de chauffage ce qui génère l'évacuation d'un gaz de chauffage (symbolisé par des triangles dans la figure 1) dans le tube de chauffage 9. Ledit gaz de chauffage vient alors descendre dans le tube de chauffage 9 avant de remonter naturellement vers l'extrémité supérieure 11 du tube de chauffage 9 où il est évacué par la conduite de sortie 15 vers l'extérieur du four de craquage 1. La présence et le mouvement du gaz de chauffage permet de chauffer efficacement le tube de chauffage 9 sur toute sa hauteur ce qui entraîne un chauffage de la zone de traitement 3 par convection (au niveau du tube de chauffage 9) et par rayonnement (de par le matériau particulier constituant l'enceinte 2). Le gaz à traiter est donc efficacement, rapidement et uniformément chauffé à la température nécessaire au craquage thermique des huiles et goudrons présents dans ledit gaz soit ici à une température d'environ 1100°C. Ceci provoque donc un craquage des éléments nuisibles tels que goudrons et/ou phases huileuses présents dans ledit gaz à traiter.

Le gaz à traiter circule naturellement, et avantageusement de manière hélicoïdal grâce à l'effet cyclonique engendré par la disposition tangentielle de l'entrée 4, dans l'enceinte 2 entre l'entrée 4 basse de l'enceinte 2 et la sortie 5 haute de l'enceinte dans toute la zone de traitement 3 ce qui lui laisse le temps d'être correctement traité avant d'être évacué de l'enceinte 2 au niveau de la sortie 5.

On constate donc que le chauffage du gaz à traiter est indirect puisqu'il n'y a pas de contact physique entre le gaz de chauffage ou le combustible et le gaz à traiter : seuls le tube de chauffage 9 et les parois internes réfractaires de l'enceinte 2 permettent de chauffer le gaz à traiter.

La configuration particulière de l'enceinte 2 et du tube de chauffage 9 associé permet ainsi de définir une zone de traitement 3 étroite dans lequel le gaz à traiter se retrouve confiné tout au long de sa traversée de l'enceinte 2, zone de traitement 3 chauffée extérieurement par les parois réfractaires internes de l'enceinte 2 et chauffée intérieurement par le tube de chauffage 9. Ceci permet d'obtenir un chauffage homogène du gaz à traiter dans toute la zone de traitement assurant ainsi un bon craquage des phases huiles et des goudrons indésirables.

En référence à la figure 2, un deuxième mode de réalisation de l'invention va être à présent décrit. Les éléments en commun avec le premier mode de réalisation conservent la même numérotation augmentée d'une centaine.

Dans ce deuxième mode de réalisation, les moyens d'évacuation des particules solides parasites ne comportent plus de conduite d'évacuation et de vanne associée mais un filtre 116 s'étendant verticalement selon l'axe X dans l'enceinte 102, coaxialement à ladite enceinte 102 et au tube de chauffage 109, de sorte que le tube de chauffage 109 s'étende à l'intérieur du filtre 116 dans l'enceinte 102. Le filtre 116 est ici également conformé de sorte à présenter une section circulaire (section ayant pour normale l'axe X). Le filtre 116 a une hauteur égale à celle de l'enceinte 102 de sorte à être solidaire d'une part du plafond 112 de l'enceinte 102 et d'autre part du fond 108 de l'enceinte.

De la sorte, l'enceinte 102 et le tube de chauffage 109 délimitent toujours entre eux un espace intérieur formant une zone de traitement 103 du gaz mais le filtre 116 et le tube de chauffage 109 délimitent en outre également entre eux une zone de filtrage 117 du gaz.

Le filtre 116 est par exemple en céramique.

L'entrée 104 de l'enceinte 102 est ici conformée pour déboucher directement dans ladite zone de filtrage 117 du gaz. A cet effet, l'entrée 104 débouche au niveau du fond 108 de l'enceinte 102 dans ladite zone de filtrage 117. Quant à la sortie 105, elle est conformée pour déboucher dans la zone de traitement 103 mais à l'extérieur de la zone de filtrage 117.

Ainsi, en service, le gaz à traiter est introduit dans l'enceinte 102 par l'entrée 104 de sorte à déboucher dans l'enceinte 102 à l'intérieur de la zone de filtrage 117. Parallèlement, le brûleur 113 assure une combustion du combustible ce qui génère l'évacuation d'un gaz de chauffage dans le tube de chauffage 109. Ledit gaz de chauffage vient alors descendre dans le tube de chauffage 109 avant de remonter naturellement vers l'extrémité supérieure 111 où il est évacué par la conduite de sortie 115 vers l'extérieur du four de craquage 101. La présence et le mouvement du gaz de chauffage permet de chauffer efficacement le tube de chauffage 109 sur toute sa hauteur ce qui entraîne un chauffage de la zone de traitement 103 par convection (au niveau du tube de chauffage 109) et par rayonnement (de par le matériau particulier constituant l'enceinte 102). Ceci provoque donc un craquage des éléments nuisibles tels que goudrons et/ou phases huileuses présents dans ledit gaz à traiter. Le gaz à traiter circule naturellement dans l'enceinte 102 entre l'entrée 104 basse de l'enceinte 102 et la sortie 105 haute de l'enceinte dans toute la zone de traitement 103. En outre, ce mouvement naturel l'oblige à traverser le filtre 116 pour remonter jusqu'à la sortie 105. Si le filtre 116 laisse passer ledit gaz, il retient en revanche les particules solides indésirables ce qui permet au gaz sortant par la sortie 105 d'être nettoyé non seulement des goudrons et des phases huileuses mais en outre des particules solides indésirables.

L'invention n'est pas limitée aux modes de réalisation décrits mais englobe au contraire toute variante entrant dans le cadre de l'invention.

Bien entendu, le four de craquage selon l'invention pourra être utilisé pour le traitement d'autres types de gaz que ceux provenant du traitement thermique de biomasses et/ou de déchets. Le four de craquage selon l'invention est cependant particulièrement adapté aux gaz provenant du traitement thermique de biomasses et/ou de déchets.

Bien qu'ici l'enceinte tubulaire présente une section circulaire, l'enceinte pourra présenter une section différente telle qu'une section elliptique. On préférera toutefois avoir une enceinte avec une section circulaire qui favorise les échanges thermiques au sein de la zone de traitement. De même le tube de chauffage pourra présenter une section différente d'une section circulaire telle qu'une section elliptique. On préférera toutefois avoir un tube de chauffage avec une section circulaire qui favorise les échanges thermiques au sein de la zone de traitement. Dans tous les cas, on privilégiera un four de craquage dans lequel le tube de chauffage et l'enceinte présentent la même forme de section ce qui favorise les échanges thermiques au sein de la zone de traitement.

Bien qu'ici l'enceinte soit en alumine, l'enceinte pourra être dans un tout autre matériau : une autre céramique, un béton réfractaire, un métal ou un alliage de métal... On privilégiera cependant les matériaux réfractaires tels que le béton réfractaire ou la céramique qui favoriseront le traitement du gaz. En outre, on prendra également en considération la nature du gaz à traiter (corrosif ou non notamment.

De même bien qu'ici le tube de chauffage soit en alumine, le tube de chauffage pourra être dans un tout autre matériau : une autre céramique, un béton réfractaire, un métal ou un alliage de métal ... On privilégiera cependant les matériaux réfractaires tels que le béton réfractaire ou la céramique qui favoriseront le traitement du gaz. En outre, on prendra également en considération la nature du gaz à traiter (corrosif ou non notamment.

De même bien qu'ici le filtre soit en alumine, le filtre pourra être dans un tout autre matériau : une autre céramique, un béton réfractaire, un métal ou un alliage de métal ... On privilégiera cependant les matériaux réfractaires tels que le béton réfractaire ou la céramique qui favoriseront le traitement du gaz. En outre, on prendra également en considération la nature du gaz à traiter (corrosif ou non notamment.

La sortie du four de craquage pourra être accordée à d'autres moyens complémentaires d'élimination des impuretés présentes dans le gaz et/ou à des moyens de purification du gaz.

## Revendications

1. Four de craquage (1 ; 101) comportant une enceinte (2 ; 102) tubulaire verticale qui comprend une entrée (4 ; 104) pour l'introduction d'un gaz à traiter et une sortie (5 ; 105) pour l'évacuation dudit gaz hors de l'enceinte, des moyens de chauffage dudit gaz qui comprennent un tube de chauffage (9 ; 109) s'étendant verticalement à l'intérieur de l'enceinte et coaxialement à l'enceinte, le tube de chauffage étant conformé de sorte à avoir son extrémité inférieure fermée et étant agencé de sorte que son extrémité inférieure soit agencée dans l'enceinte et de sorte que son extrémité supérieure soit raccordée à un brûleur (13 ; 113) des moyens de chauffage qui est agencé à l'extérieur de l'enceinte, dans lequel l'entrée (4) est agencée de sorte à faire pénétrer le gaz à traiter dans l'enceinte (2) le long de la paroi interne de l'enceinte (2) afin de générer un effet cyclonique de sorte que le gaz s'écoule hélicoïdalement dans l'enceinte.

2. Four de craquage (1 ; 101) selon la revendication 1, dans lequel l'enceinte (2 ; 102) est conformée de sorte à avoir une section circulaire.

3. Four de craquage (1 ; 101) selon la revendication 1 ou 2, dans lequel le tube de chauffage (9 ; 109) est conformé de sorte à avoir une section circulaire.

4. Four de craquage (1 ; 101) selon l'une des revendications 1 à 3, dans lequel le tube de chauffage (9 ; 109) est à base de céramique.

5. Four de craquage (1 ; 101) selon l'une des revendications 1 à 4, dans lequel au moins les parois internes de l'enceinte (2 ; 102) sont en matériau réfractaire.

6. Four de craquage (1 ; 101) selon la revendication 5, dans lequel le matériau réfractaire est à base de céramique.

7. Four de craquage (1 ; 101) selon la revendication 6, dans lequel la céramique comporte de l'alumine.

8. Four de craquage (1 ; 101) selon la revendication 5, dans lequel le matériau réfractaire est à base de béton.

9. Four de craquage (1 ; 101) selon l'une des revendications 1 à 8, comportant des moyens d'évacuation de particules solides parasites contenues dans le gaz à traiter.

10. Four de craquage (1) selon la revendication 9, dans lequel lesdits moyens d'évacuation comportent une conduite d'évacuation (6) s'étendant depuis un fond (8) de l'enceinte (2) vers l'extérieur de l'enceinte et une vanne (7) agencée dans ladite conduite d'évacuation.

11. Four de craquage (1) selon la revendication 10, dans lequel le fond (8) de l'enceinte (2) est concave de sorte à former un entonnoir.

12. Four de craquage (101) selon la revendication 9, dans lequel lesdits moyens d'évacuation comportent un filtre (116) s'étendant verticalement dans l'enceinte (102), coaxialement à ladite enceinte (102) et au tube de chauffage (109), de sorte que le tube de chauffage s'étende à l'intérieur du filtre dans l'enceinte.

13. Four de craquage (101) selon la revendication 12, dans lequel le filtre (116) est à base de céramique.

14. Four de craquage (1 ; 101) selon l'une des revendications 1 à 13, dans lequel les moyens de chauffage sont conformés pour prélever une partie du gaz au niveau de la sortie (5 ; 105) du four de craquage pour alimenter le brûleur (13 ; 113).

15. Ensemble d'un four de craquage (1 ; 101) selon l'une des revendications 1 à 14 et d'un dispositif de traitement thermique de biomasses et/ou de déchets dont une sortie est raccordée à l'entrée (4 ; 104) du four de craquage.

16. Ensemble selon la revendication 15, dans lequel le dispositif de traitement thermique comporte un bâti et des moyens de convoyage des biomasses et/ou des déchets entre l'entrée et la sortie du bâti qui comprennent une vis montée pour tourner dans le bâti selon un axe géométrique de rotation et des moyens d'entraînement en rotation de la vis, le dispositif comportant en outre des moyens de chauffage de la vis par effet Joule.
